# EUROPEAN PATENT SPECIFICATION

(11) **EP 0 599 354 B1**
(45) Date of publication and mention of the grant of the patent: **29.04.1998**
(21) Application number: 93119774.3
(22) Date of filing: 19.10.1987
(51) Int. Cl.: A61B 17/34

(54) **A trocar**
Ein Trokar
Un trocart

(30) Priority: 17.10.1986 US 920509
(43) Date of publication of application: 01.06.1994
(62) Divisional of application: 87309189.6
(73) Proprietor: United States Surgical Corporation, Norwalk, Connecticut 06856 (US)
(72) Inventor: Lander, Jack R., Danbury, CT 06811 (US); Holmes, William A., Marblehead, MA 01945 (US); Costa, Peter F., Sommerville, Mass. 02143 (US)
(74) Representative: Marsh, Roy David

(56) References cited:
- GB-A- 904 635
- GB-A- 1 356 386
- US-A- 2 925 083
- US-A- 4 601 710
- US-A- 4 654 030

## Description

This invention relates to a trocar. More particularly, this invention relates to a latch means for a trocar.

As is known, a trocar is generally made of a stylet having a sharp tip for penetrating through an abdominal wall and a surrounding tube through which gas or the like may be injected or exhausted from the abdomen after puncturing by the stylet. Further, in order to protect a patient or surgeon from injury through inadvertant contact with the sharp tip, protective tubes have been positioned over the stylet tip, for example, as described in U.S. Patent 4,601,710. In this case, the stylet is secured to a housing with a spring-biased protective tube concentrically about the stylet. When put to use, the protective tube is able to move proximally to expose the sharp tip for penetrating the abdominal wall without interference. Once a puncture has been made, the protective tube is able to slide distally under the force of the spring to again cover over the sharp tip to preclude injury to tissue within the abdomen. Thereafter, the stylet can be removed from the abdomen.

In addition, the known trocar has been constructed so that the housing secured to the stylet can be fitted into and rotated relative to a second housing containing an outer tube through which the stylet and protective tube may pass. Further, the second housing has had a flap valve which cooperates with a groove in the protective tube in order to lock the spring-biased protective tube in an extended position over the sharp stylet tip. Generally, the trocar is delivered for use in this locked condition so as to protect the user from being injured by the sharp tip of the stylet. However, prior to use, the housing connected to the stylet must be pushed into an unlocked position so that the protective tube can retract from the sharp tip in order to puncture an abdominal wall.

In the event that the trocar is not delivered in the locked position, there is a risk that users may be damaged should the protective tube inadvertently be retracted to expose the sharp stylet tip. Further, any exposed tissue of a patient may be damaged should the protected tube be retracted inadvertently prior to proper positioning of the stylet for penetrating an abdominal wall.

Accordingly, it is an object of the invention to provide a trocar in which the risk of injury due to inadvertent retraction of a protective tube over a sharp stylet tip can be significantly reduced.

It is another object of the invention to shield the sharp tip of a stylet of a trocar in a positive manner at all times prior to actual use.

It is another object to the invention to provide a relatively simple structure for shielding the sharp tip of a trocar until ready for use.

It is another object to the invention to provide a relatively simple mechanism for unlocking a trocar for use.

Briefly, the invention provides a trocar of two part construction.

The trocar is formed of a first housing, a stylet which is secured to and which extends from the first housing with a sharp tip at a distal end, a protective member which is slidably mounted in the first housing between an extended position and a retracted position with the protective member disposed about the stylet and a spring in the first housing for biasing the protective member outwardly of the first housing into the extended position. The protective member further includes three fingers at its distal end. In accordance with the invention, a latch means is provided in the first housing for locking the protective member in the extended position and a slider is mounted in and projects from the first housing for releasing the latch means to permit movement of the protective member into the retracted position.

The trocar further comprises a second housing which is coaxial with the first housing and a guide tube which is secured to and which extends from the second housing with the protective member slidably receivable therein. The second housing is also provided, in known manner, with a seal ring through which the protective member slides in seal-tight manner and a flap valve which covers over the seal ring when the first part has been removed from the second part of the trocar.

The second housing preferably defines a handgrip.

When in use, the two housings are disposed in face-to-face manner with the slider of the first housing projecting against the second housing. In this position, the protective member is retained in a locked position by the latch means. However, when the user is ready to penetrate, for example through an abdominal wall of the patient, the two housings are squeezed together so as to push the slider into the first housing to release the latch means. At this time, the protective member is unlatched so as to be slid proximally during penetration through an abdominal wall as is know. However, once penetration has been completed, the protective member is spring-biased distally into the extended position and reengaged by the latch means so as to be locked in place. The stylet may then be removed from the abdomen while the protective member remains in a locked protective position about the sharp tip of the stylet.

The latch means includes a leaf spring which abuts the protective member in the extended position as well as a spring finger which extends from the leaf spring into abutment with the slider for lifting of the leaf spring from the protective member in response to movement of the slider into the housing. In this regard, a collar is provided on the protective member to abut the leaf spring spring when in the extended position. In addition, the collar is slidable along the leaf spring in order to lift and release the spring finger from the slider at an intermediate point of movement along the leaf spring. Thus, once the slider has released the latch means, and the protective member has moved a certain distance, the slider is no longer active so that any return of the protective member to the extended position automatically activates the latch means into the locked position preventing further proximal movement of the protective member.

An indicator may also be secured to the protective member for exposure through the first housing to indicate the position of the protective member relative to the stylet tip during use.

Preferably, the slider projects from the first housing less in the second position than in the first, the movement of the slider from the first to the second position corresponding to a depression of the slider relative to the first housing.

These and other objects and advantages of the invention will become more apparent from the following description taken in conjunction with the accompanying drawings wherein:
Fig. 1 illustrates a perspective view of a trocar constructed in accordance with the invention.
Fig. 2 illustrates a cross-sectional view of the trocar of Fig. 1 in a locked position;
Fig. 3 illustrates a perspective view of a latch constructed in accordance with the invention;
Fig. 4 illustrates a partial cross-sectional view of the trocar in a position in which the protective member in this embodiment a protective tube has been initially released for penetration of an abdominal wall;
Fig. 5 illustrates a view similar to Fig. 4 with the spring finger released from the slider;
Fig. 6 illustrates a view similar to Figs. 4 and 5 with the latch means returned to a locked position with the protective tube in an extended position; and
Fig. 7 illustrates a part perspective view of the latch means and slider of the trocar in a locked position.

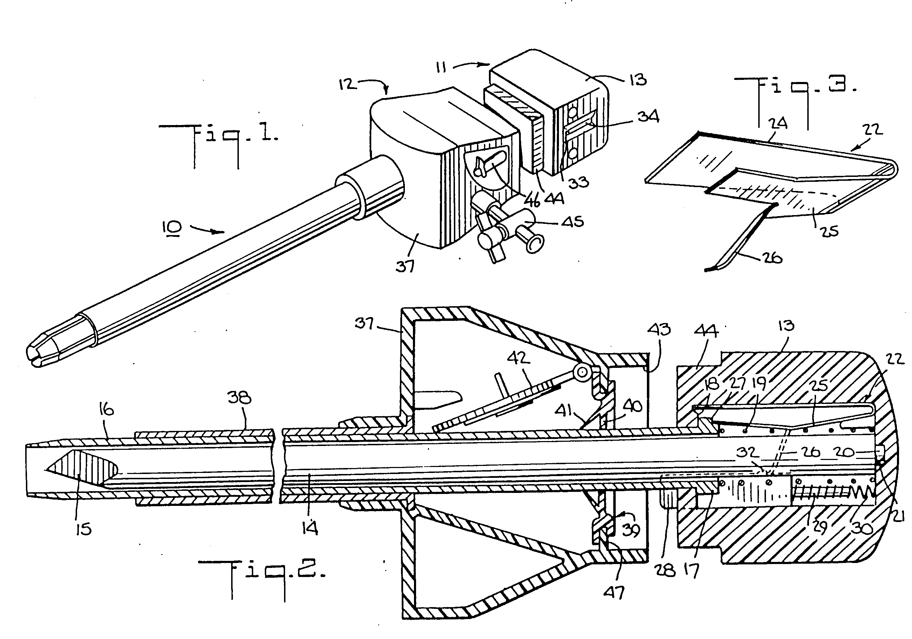

Referring to Fig. 1, the trocar 10 is constructed of two parts 11, 12 which are arranged in a coaxial manner.

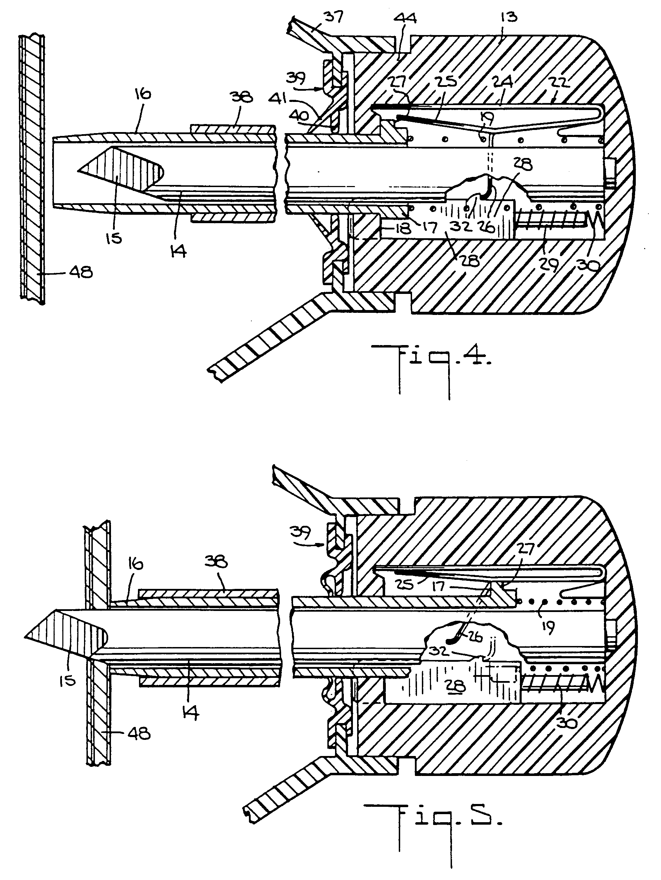

Referring to Figs. 1 and 2, one part 11 includes a housing 13, for example made of plastic and a stylet 14, for example made of steel, which is secured to and extends from the housing 13 with a sharp tip 15, as is known, at the distal end. In addition, a protective tube 16, for example made of steel or aluminum is slidably mounted in the housing 13 between an extended position, as viewed in Fig. 2, and a retracted position while being disposed concentrically about the stylet 14. The tube 16 carries a collar 17 at the proximal end within the housing 13 to abut against a wall 18 of the housing under the bias of a coil spring 19. As indicated, the spring 19 is concentric to the proximal end of the stylet 14 and abuts against a back wall of the housing 13. In addition, the stylet 14 has a reduced portion 20 which is disposed within a recess 21 in the back wall of the housing 13 and secured in place, for example by an adhesive or other suitable means.

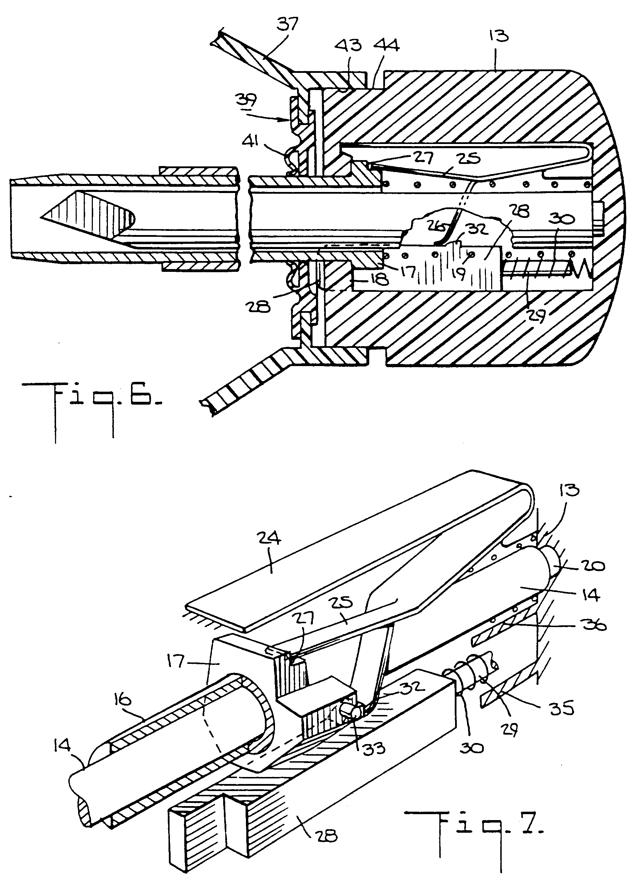

Referring to Figs. 2 and 3, a latch means 22 is disposed in the housing 13 for locking the protective tube 16 in the extended position illustrated. As indicated in Fig. 3, the latch means 22 is in the form of a one-piece body having a flat base 24, a leaf spring 25 which extends from one end of the base 24 to define a U-shape and a spring finger 26 which extends angularly and intermediately from the leaf spring 25. As shown, the leaf spring 24 has a proximal portion of a width equal to the width of the base 22 which extends from the base 22 to an intermediate point and a distal portion of reduced width extending to the distal end. The spring finger 26 extends from this intermediate point.

As indicated in Figs. 2 and 7, the reduced portion of the leaf spring 25 engages against the collar 17 on the protective tube 16. To this end, the collar 17 has a shoulder 27 at the upper end, as viewed, against which the end of the leaf spring 26 abuts. When the leaf spring 27 is in the position shown the protective sleeve 16 is prevented from moving proximally. As indicated in Fig. 2, the base 22 of the latch means is retained within the housing 13 in a fixed manner so as to be restrained against movement proximally or distally.

Referring to Fig. 2, a slider 28 is slidably mounted in and projects from the housing 13 for releasing the latch means 22 in order to permit movement of the protective tube 16 into a retracted position. The slider 28 is made of any suitable material, such as a metal or plastic and includes a stem 29 at the proximal end about which a spring 30 is disposed for biasing the slider 28 in a distal direction. As indicated, the spring 30 abuts against the back wall of the housing 13 while the slider has a shoulder portion 31 (see Fig. 7) for abutting against a front wall of the housing 13 in an extended position thereof. The slider 28 also has a shoulder 32 on an upper surface, as viewed, for engaging the free end of the spring finger 26. The shoulder 32 acts in the manner of a cam or single tooth ratchet so that when the slider 28 is moved into the housing 13 against the bias of the spring 30, the spring finger 26 pivots in a counter-clockwise manner as viewed while also lifting the leaf spring 25 from the collar 17. to release the protective tube 16 (see Fig. 4). Alternatively, other means such as a recess in the slider 28 can be used to accomplish the engagement of the spring finger 26. At this time, the tube 16 is freed to be able to move proximally, for example as indicated in Fig. 5. During this time, the collar 17 slides along the leaf spring 25 causing the leaf spring 25 to move towards the base 22 thereby lifting and releasing the spring finger 26 from the shoulder 32, for example at an intermediate point of movement of the collar 17 along the leaf spring 25.

Referring to Fig. 7, the collar 17 also carries a guide pin 33 which extends radially outwardly of the protective sleeve 16. As indicated in Fig. 1, this guide pin 33 is received in a guide slot 34 in a side wall of the housing 13. The guide pin 13 and slot 34 thus serve to retain the collar 17 in alignment with the leaf spring 25 during use. In addition, the pin 33 serves as an indicator to indicate the position of the protective tube 16 relative to the tip 15 of the stylet 14. For example, the distal most position (Fig. 1) indicates the tube 16 is in the extended position. A surgeon can thus determine where the tube 16 is during a surgical procedure.

Referring to Fig. 7, the housing 13 may be provided with a stop portion 35 for abutting the proximal end of the slider 28 and thus establish a rear most position of the slider 28. In similar manner, the housing may be provided with a stop portion 36 for abutting the collar 17 so as to provide a rear most position for the protective tube 16.

Referring to Figs. 1 and 2, the second part 12 of the trocar 10 is constructed in conventional manner. For example, this part 12 includes a housing 37, for example of plastic and a guide tube 38 which is secured to and extends from the housing 37 so as to slidably mount the protective tube therein. In addition, a seal ring 39 is mounted within the housing 37 about an opening through which the protective tube 16 of the trocar part 11 passes. This seal ring 39 includes a main portion 40 which sealingly engages the outer peripheral of the protective tube 16 and an annular flap 41 which extends from the main portion 40 to sealingly engage the protective tube 16 at a point distal from the main portion 40. During retraction of the protective tube 16, the seal flap 41 tends to deform in an S-shape manner as indicated in Fig. 5.

The housing 37 also has a flap valve 42 pivotally mounted therein in known manner for sealing over the seal ring 39 after the trocar part 11 has been removed.

Referring to Figs. 1 and 2, the housing 37 of the trocar part 12 is provided with a rectangular recess 43 at the proximal end while the housing 13 of the trocar part 11 is provided with a reduced portion 44 of rectangular shape which is sized to fit within the recess 43.

In addition, the housing 37 is provided with a valve construction 45 as well as a pointer 46 to indicate the position of the flap valve 42.

In order to use the trocar 10, the two housing parts 11, 12 are placed together. For example, as indicated in Fig. 2, the protective tube 16 is slid through the ring seal 40 with the flap valve resting against the outer periphery of the protective tube 16. At this time, the leaf spring 25 engages against the shoulder 27 of the collar 17 so as to lock the protective tube 16 in the extended position. Also, the slider 28 projects out of the distal face of the housing 13.

Next, as indicated in Fig. 4, the two housings 13, 37 are brought together so that the reduced portion 44 of the housing 13 moves into the recess 43 of the housing 12 with the slider 28 abutting a rear wall 47 of the housing 37. Next, the user squeezes the two housings 13, 37 together so as to move the slider 28 proximally into the housing 13. In this regard, the two housings 13, 37 function as a handgrip for the user.

As indicated in Fig. 4, when the slider has moved into the housing 13, the spring finger 26 is pivoted in a counter-clockwise manner via the shoulder 32 on the slider 28 while also moving upwardly to lift the leaf spring 25 from the shoulder 27 of the collar 17. In the position shown, the protective tube 16 is free to move relative to the stylet 14. The user then penetrates through the abdominal wall 48 of a patient in known manner so that the sharp tip 15 of the stylet 14 punctures the wall 48 as indicated in Fig. 5. During this initial phase, the protective tube 16 moves proximally within the guide tube 38 with the collar 17 sliding along the leaf spring 25. At an intermediate point, for example where the finger 26 extends from the leaf spring 25, the collar deflects the leaf spring 25 upwardly causing the spring finger 26 to lift above the shoulder 32 on the slider 28. The spring finger 26 then pivots in a clockwise manner into the full line position illustrated in Fig. 5. The protective tube 16 may slide further proximally until abutting the stop 36 (see Fig. 7).

Of note, the stylet 14, protective tube 16 and guide tube 38 are sized so that the rear most retraction of the protective tube 16 places the distal end of the protective tube 16 slightly behind the sharp tip 15 of the stylet 14 but ahead of the guide tube 38. In addition, the respective diameters of the stylet 14 and tube 16, 38 are such that a slide fit relationship is presented so that both tubes 16, 38 may follow the sharp tip 15 through the wall 48 of the patient into the interior of the abdomen, as is known.

After the protective tube 16 passes through the final layer of the abdominal wall 48 and the sharp tip 15 is within an inflated cavity between the abdominal wall 48 and other organs, which cavity has been intentionally inflated, the spring 19 pushes the protective tube 16 outwardly to the extended position indicated in Fig. 6. At this time, the leaf spring 25 re-engages the shoulder 27 of the collar 17 to lock the protective tube 16 in place over the stylet tip 15. As indicated, the spring finger 26 is on the distal side of the shoulder 32 of the slider 28 and cannot be triggered again unless returned to the position indicated in Fig. 2.

The user may then release the housings 13, 37. Next, for example, the trocar part 11 may be slid out of the trocar part 12 with the flap valve 42 closing over the ring seal 40. This component 12 may then be used in a conventional manner. The removed component 11, on the other hand, has the guide tube 16 locked in place over the stylet tip 15 so as to protect against injury from the sharp edges of the tip 16.

In the event that the trocar 10 would require a second triggering of the latch means 22, the trocar part 11 can be slid slightly out of the housing 37 so that the slider 28 again projects out of the housing 13 so that the spring finger 26 engages behind the shoulder 32. A further squeezing action can then be carried out to again release the guide tube 16 in the manner described above.

The invention thus provides a trocar which can be easily manipulated by a user while the sharp tip of the stylet is maintained in a protective environment by the protective tube.

Further, the invention provides a trocar in which the protective tube is maintained in a locked position relative to the stylet tip until actual penetration through an abdominal wall or the like is required.

Of note, instead of using a latch means wherein a base and a leaf spring define a U-shape, the latch means may simply constitute a leaf spring mounted directly to the housing in a cantilevered manner with the spring finger depending therefrom.

## Claims

1. A trocar comprising:
a first housing (13);
a stylet (14) secured to and extending from said first housing, said stylet having a sharp tip (15) at a distal end;
a protective member (16) slidably mounted in said first housing between an extended and a retracted position, said protective member including a distal end including three fingers, the distal end being disposed with respect to said stylet when in said extended position to protect against injury from said sharp tip;
a spring (19) in said first housing biasing said protective member outwardly of said first housing into said extended position; and
a second housing (37) coaxial with said first housing (13) and a guide tube (38) secured to and extending from said second housing with said protective member (16) slidably receivable therein;
the trocar being **characterised by**:
latch means (22) in said first housing for locking said protective member in said extended position; and
a slider (28) mounted in and projecting from said first housing, for releasing said latch means.

2. A trocar as claimed in claim 1 which further comprises a collar (17) on said protective member wherein said latch means includes leaf spring (25) which, in a first non-deflected position is in engagement with said collar to resist movement of the protective member from the extended position towards the retracted position, and a spring finger (26) itself actuated by the slider (28) for lifting said leaf spring from its first, non-deflected position to a second, deflected position to permit movement of the protective member.

3. A trocar as claimed in claim 2, wherein movement of said protective member from said retracted position to said extended position permits the leaf spring to return to its non-deflected position.

4. A trocar as claimed in any one of the preceding claims which further comprises a seal ring (39) in said second housing having a main portion (40) sealingly engaging said protective member (16) and an annular flap (41) extending from said main portion and sealingly engaging said protective member (16).

5. A trocar as claimed in any one of the preceding claims, wherein said slider (28) mounted in and projecting from said first housing (13) contacts said second housing (37) upon squeezing of said housings together, thereby causing said slider (28) to move into said first housing (13) to release said latch means (22).

6. A trocar as claimed in any one of the preceding claims, wherein said latch means is adapted to lock said protective member in said extended position upon a return movement of said protective member from said retracted position to said extended position.

7. A trocar as claimed in any one of the preceding claims, which further comprises a guide slot (34) in said first housing and a guide pin (33) secured to said protective member (16) slidably mounted in said slot.

8. A trocar as claimed in any one of the preceding claims, wherein the slider is elongate and has a proximal end and a distal end, and slides between a first position and a second position.

9. A trocar as claimed in claim 8, wherein the slider (28) projects from the first housing (13) less in the second position than in the first position, the movement of the slider from the first position to the second position corresponding to a depression of the slider relative to the first housing.

## Patentansprüche

1. Trokar umfassend:
ein erstes Gehäuse (13;)
ein Mandrin (14), der an dem ersten Gehäuse befestigt ist und sich von diesem erstreckt, wobei der Mandrin eine scharfe Spitze (15) an einem distalen Ende besitzt;
ein Schutzelement (16), das in dem Gehäuse zwischen einer ausgestreckten und einer zurückgezogenen Position verschiebbar befestigt ist, wobei das Schutzelement ein distales Ende mit drei Fingern umfaßt und das distale Ende in Bezug auf den Mandrin, wenn dieser in der ausgestreckten Position ist, angeordnet ist, um gegen eine Verletzung durch die scharfe Spitze zu schützen;
eine Feder (19) in dem ersten Gehäuse, die das Schutzelement nach außen aus dem ersten Gehäuse in die ausgestreckte Position vorspannt; und
ein zweites Gehäuse (37) koaxial mit dem erste Gehäuse (13) und eine Führungsröhre (38), die an dem zweiten Gehäuse befestigt ist und sich von diesem erstreckt, wobei das Schutzelement (16) verschiebbar darin aufnehmbar ist;
wobei der Trokar gekennzeichnet ist durch:
eine Verriegelungseinrichtung (22) in dem ersten Gehäuse, um das Schutzelement in der ausgestreckten Position zu verriegeln; und
ein Schiebeelement (28), das in dem ersten Gehäuse befestigt ist und sich von diesem erstreckt, um die Verriegelungseinrichtung zu lösen.

2. Trokar gemäß Anspruch 1, weiter umfassend:
einen Kragen (17) auf dem Schutzelement, wobei die Verriegelungseinrichtung eine Blattfeder (25) umfaßt, die, in einer ersten, nicht abgebogenen Posititon, im Eingriff mit dem Kragen ist, um einer Bewegung des Schutzelementes von der ausgestreckten Position in Richtung der zurückgezogenen Position zu widerstehen, und einen Federfinger (26), der selbst durch das Schiebeelement (28) betätigt wird, um die Blattfeder von ihrer ersten, nicht abgebogenen Position in eine zweite, abgebogene Position anzuheben, um eine Bewegung des Schutzelementes zu gestatten.

3. Trokar gemäß Anspruch 2, wobei die Bewegung des Schutzelementes von der zurückgezogenen Position in die ausgestreckte Position es der Blattfeder gestattet, in ihre nicht abgebogene Position zurückzukehren.

4. Trokar gemäß einem der vorhergehenden Ansprüche, weiter umfassend einen Abdichtring (39) in dem zweiten Gehäuse mit einem Hauptbereich (40), der dicht in Eingriff tritt mit dem Schutzelement (16) und eine ringförmige Klappe (41), die sich von dem Hauptbereich erstreckt und dicht in Eingriff tritt mit dem Schutzelement (16).

5. Trokar gemäß einem der vorhergehenden Ansprüche, wobei das Schiebeelement (28), das in dem ersten Gehäuse (13) befestigt ist und von diesem vorsteht, in Kontakt ist mit dem zweiten Gehäuse (37) auf das Zusammendrücken der beiden Gehäuse beim, was verursacht, daß sich das Schiebeelement (28) in das erste Gehäuse (13) bewegt, um die Verriegelungseinrichtung (22) zu lösen.

6. Trokar gemäß einem der vorhergehenden Ansprüche, wobei die Verriegelungseinrichtung dazu angepaßt ist, das Schutzelement in der ausgestreckten Position auf eine Rückkehrbewegung des Schutzelementes von der zurückgezogenen Position in die ausgestreckte Position hin zu verriegeln.

7. Trokar gemäß einem der vorhergehenden Ansprüche, weiter umfassend: einen Führungsschlitz (34) in dem ersten Gehäuse und einen Führungsstift (33), der an dem Schutzelement (16) befestigt ist, der verschiebbar in dem Schlitz montiert ist.

8. Trokar gemäß einem der vorhergehenden Ansprüche, wobei das Schiebeelement langgestreckt ist und ein proximales Ende und ein distales Ende besitzt, und sich zwischen einer ersten Position und einer zweiten Position verschiebt.

9. Trokar gemäß Anspruch 8, wobei das Schiebeelement (28) vom ersten Gehäuse (13) in der zweiten Position weniger als in der ersten Position hervorsteht, wobei die Bewegung des Schiebeelementes von der ersten Position zu der zweiten Position einem Niederdrücken des Schiebeelementes relativ zum ersten Gehäuse entspricht.

## Revendications

1. Trocart comprenant :
un premier boitier (13) ;
un stylet (14) fixé à et s'étendant dudit premier boîtier, ledit stylet ayant une pointe tranchante (15) à une extrémité distale ;
un élément de protection (16) installé de manière coulissante dans ledit premier boîtier entre des positions étendue et rétractée, ledit élément de protection incluant une extrémité distale comprenant trois doigts, l'extrémité distale étant disposée relativement audit stylet, lors de ladite position étendue, pour protéger contre une blessure par ladite pointe tranchante ;
un ressort (19) dans ledit premier boîtier sollicitant ledit élément de protection vers l'extérieur dudit premier boîtier dans ladite position étendue ; et
un deuxième boîtier (37) coaxial avec ledit premier boîtier (13) et un tube de guidage (38) fixé à et s'étendant dudit deuxième boîtier, ledit élément de protection (16) pouvant être reçu de manière coulissante dans celui-ci ;
le trocart étant caractérisé par :
un moyen formant loquet (22) dans ledit premier boîtier pour verrouiller ledit élément de protection dans ladite position étendue ; et
une pièce coulissante (28) installée dans et dépassant dudit premier boîtier pour libérer ledit moyen formant loquet.

2. Trocart selon la revendication 1, qui comprend en outre un collier (17) sur ledit élément de protection, où ledit moyen formant loquet comporte un ressort à lames (25) qui, dans une première position non déviée, est en prise avec ledit collier pour résister à un déplacement de l'élément de protection de la position étendue vers la position rétractée, et un doigt de ressort (26) actionné lui-même par la pièce coulissante (28) pour relever ledit ressort à lames de sa première position non déviée à une deuxième position déviée pour permettre le déplacement de l'élément de protection.

3. Trocart selon la revendication 2, où le déplacement dudit élément de protection de ladite position rétractée à ladite position étendue permet au ressort à lames de retourner à sa position non déviée.

4. Trocart selon l'une des revendications précédentes, qui comporte en outre une bague d'étanchéité (39) dans ledit deuxième boîtier ayant une portion principale (40) mise en prise étanche avec ledit élément de protection (16) et un volet annulaire (41) s'étendant de ladite portion principale et venant en prise étanche avec ledit élément de protection (16).

5. Trocart selon l'une des revendications précédentes, où ladite pièce coulissante (28) montée dans et faisant saillie dudit premier boîtier (13) vient en contact avec ledit deuxième boîtier (37) lors du serrage desdits boîtiers ensemble en amenant ainsi ladite pièce coulissante (28) de rentrer dans ledit premier boîtier (13) pour libérer ledit moyen formant loquet (22).

6. Trocart selon l'une des revendications précédentes, où ledit moyen formant loquet est apte à verrouiller ledit élément de protection dans ladite position étendue lors d'un mouvement de retour dudit élément de protection de ladite position rétractée à ladite position étendue.

7. Trocart selon l'une des revendications précédentes, qui comporte en outre une fente de guidage (34) dans ledit premier boitier et un axe de guidage (33) fixé audit élément de protection (16) installé de manière coulissante dans ladite fente.

8. Trocart selon l'une des revendications précédentes, où la pièce coulissante est oblongue et a une extrémité proximale et une extrémité distale et coulisse entre une première position et une deuxième position.

9. Trocart selon la revendication 8, où la pièce coulissante (28) fait saillie du premier boîtier (13) moins dans la deuxième position que dans la première position, le déplacement de la pièce coulissante de la première position à la deuxième position correspondant à un enfoncement de la pièce coulissante relativement au premier boîtier.
